# EUROPÄISCHE PATENTSCHRIFT

(11) **EP 2 753 276 B1**
(45) Veröffentlichungstag und Bekanntmachung des Hinweises auf die Patenterteilung: **08.03.2017**
(21) Anmeldenummer: 12741020.7
(22) Anmeldetag: 02.08.2012
(51) Int. Cl.: A61F 13/02, A61M 27/00, A61M 1/00

(54) **ANSCHLUSSVORRICHTUNG ZUR VERWENDUNG BEI DER UNTERDRUCKBEHANDLUNG VON WUNDEN**
CONNECTION DEVICE FOR USE IN NEGATIVE PRESSURE WOUND THERAPY
DISPOSITIF DE RACCORDEMENT À UTILISER POUR LE TRAITEMENT SOUS DÉPRESSION DE PLAIES

(30) Priorität: 08.09.2011 DE 102011082341
(43) Veröffentlichungstag der Anmeldung: 16.07.2014
(73) Patentinhaber: Paul Hartmann AG, 89522 Heidenheim (DE)
(72) Erfinder: CROIZAT, Dr. Pierre, 89542 Herbrechtingen (DE); ECKSTEIN, Dr. Axel, 89522 Heidenheim (DE); WINGENDER, Julia, 88662 Ueberlingen (DE)
(74) Vertreter: DREISS Patentanwälte PartG mbB
(86) Internationale Anmeldenummer: PCT/EP2012/065132
(87) Internationale Veröffentlichungsnummer: WO 2013/034374

(56) Entgegenhaltungen:
- WO-A1-2008/011774
- WO-A1-2009/021353
- DE-A1- 4 012 232
- US-A1- 2006 100 586
- US-A1- 2009 054 855
- US-A1- 2009 275 884
- US-A1- 2011 152 800

## Beschreibung

Die Erfindung betrifft eine Sachgesamtheit zur Verwendung bei der Unterdruckbehandlung von Wunden aus einer Anschlussvorrichtung, einem Unterdruckverband und einer, insbesondere ein Schaummaterial aufweisenden Wundauflage auf der wundzugewandten Seite des Unterdruckverbands, wobei die Anschlussvorrichtung mit einer mit Unterdruck beaufschlagbaren Saugleitung und mit einem flächenhaften unterdruckdichten und biegsamen Trägermittel für die Saugleitung ausgebildet ist, an dem die Saugleitung unterdruckdicht gehalten ist, wobei das Trägermittel auf einen die Wunde überfangenden und gegen die Atmosphäre dichtend abschließenden Unterdruckverband im wesentlichen unterdruckdicht aufbringbar ist, so dass die Saugleitung durch wenigstens eine Öffnung in dem Trägermittel und in dem Unterdruckverband hindurch mit dem Wundraum kommunizieren kann, wobei die Saugleitung biegsam und flach ausgebildet ist und in einem wundseitigen Längsabschnitt mit wenigstens 70 % ihrer senkrecht auf das Trägermittel projizierten Fläche mit dem Trägermittel für den bestimmungsgemäßen Gebrauch unlösbar und flächenhaft verbunden ist, wobei das Trägermittel und die Saugleitung mit ihren aus ihrer flachen Form resultierenden Hauptebenen zueinander parallel sind, und wobei die Dickenerstreckung (D) des Verbunds aus Saugleitung und Trägermittel höchstens 7 mm beträgt.

In der jüngeren Vergangenheit hat die Unterdruckbehandlung von Wunden, insbesondere von tiefen und daher a priori problematisch heilenden Wunden, eine zunehmende Bedeutung erlangt. Dabei bedeutet Unterdruckbehandlung, dass ein an sich der umgebenden Atmosphäre ausgesetzter Körper- oder Wundbereich durch noch näher zu beschreibende Mittel druckdicht bzw. unterdruckdicht gegen die Umgebung, also die Atmosphäre in der wir leben und atmen, abgeschlossen wird, wobei innerhalb des abgeschlossenen Wundbereichs auf ebenfalls noch zu erläuternde Weise ein gegenüber dem Atmosphärendruck verringerter Druck, mithin also Unterdruck relativ zur Atmosphäre angelegt und dauerhaft aufrechterhalten werden kann. Wenn auf dem hier in Rede stehenden Gebiet von Unterdruck die Rede ist, so wird hiermit ein Druckbereich verstanden, der typischerweise zwischen 0 und 250 mmHg (mm Quecksilbersäule) unterhalb des umgebenden Atmosphärendrucks liegt. Es hat sich gezeigt, dass dies der Wundheilung förderlich ist. Für den unterdruckdichten Abschluss wird ein Unterdruckverband vorgesehen, der beispielsweise eine druck- bzw. unterdruckdichte Folienschicht umfassen kann, die typischerweise mit einem die Wunde umgebenden unversehrten Körperbereich verklebt ist, so dass auf diese Weise ein dichter Abschluss erreicht werden kann. Um ausgehend von einer Unterdruck erzeugenden Einrichtung, also einer Unterdruckpumpe im weitesten Sinn, einen Unterdruck an den Wundraum heranzuführen und dort aufrechtzuerhalten, können bei hier in Rede stehenden Systemen zur Unterdrucktherapie von Wunden mit Unterdruck beaufschlagbare Leitungsmittel verwendet werden, die mittels einer Anschlussvorrichtung mit dem Unterdruckverband zusammenwirken, um Unterdruck an bzw. in den Wundraum zu bringen.

Eine Anschlussvorrichtung und Sachgesamtheit der eingangs erwähnten Art ist aus DE 10 2009 060 596 A1 bzw. der entsprechenden US 2011/0152800 A1 bekannt. US 2006/0100586 A1 zeigt eine nicht gattungsgemäße Sachgesamtheit mit einer Anschlussvorrichtung mit zwei aus Rundschläuchen gebildeten Leitungen, von denen eine als Saugleitung und die andere als Messleitung oder Fluidzuführleitung dient.

Der vorliegenden Erfindung liegt die Aufgabe zugrunde, eine Anschlussvorrichtung der eingangs beschriebenen Art zu schaffen, bei der eine vielfältigere Verwendung und Therapie möglich ist.

Diese Aufgabe wird durch eine Sachgesamtheit mit den Merkmalen des Anspruchs 1 gelöst.

Es wird also erfindungsgemäß vorgeschlagen, eine zusätzliche Fluidzuführleitung vorzusehen, welche sich durch die Anschlussvorrichtung hindurch erstreckt, derart, dass auf der wundzugewandten Seite des Trägermittels ein Abschnitt der Fluidzuführleitung vorhanden ist, der zur direkten Einleitung in den Wundraum oder in eine dort vorgesehene Wundauflage genutzt werden kann, um so eine Fluideinleitung direkt in den Wundraum zu realisieren. Auf diese Weise lässt sich die Anschlussvorrichtung zu einer Unterdruckbehandlung einsetzen, im Zuge derer kontinuierlich oder diskontinuierlich ein Fluid, insbesondere eine Flüssigkeit oder ein Gas, wie z.B. Sauerstoff, insbesondere mit pharmazeutisch wirksamen und die Wundheilung unterstützenden Wirkstoffen, dem Wundraum zugeführt werden kann. Die Anschlussvorrichtung kann also zur sogenannten Instillationstherapie eingesetzt werden. Das über die Fluidzuführleitung zuführbare Fluid kann auch antimikrobiell oder analgetisch wirken. Es kann sich hierbei aber auch lediglich um eine Spüllösung, wie insbesondere eine Ringerlösung, handeln. Dadurch, dass die Fluidzuführleitung erst unterhalb des Trägermittels in den Wundraum mündet, kann sichergestellt werden, dass die zuzuführende Flüssigkeit auch wirklich in den Wundraum und vorzugsweise tief in den Wundraum und weiter vorzugsweise bis zum Wundgrund gelangt und dort ihre intendierte Wirkung ausüben kann. Die zugeführte Flüssigkeit und gegebenenfalls zusätzliche Wundflüssigkeiten oder Wundsekrete werden in an sich bekannter Weise über die Saugleitung abgeführt und in Richtung auf eine Unterdruck erzeugende Einrichtung gesogen. Vorrichtungen zur Bereitstellung von Unterdruck für die Unterdruckbehandlung von Wunden, die neben einer Unterdruck erzeugenden Einrichtung auch einen nach Gebrauch vorzugsweise wegwerfbaren Einweg-Behälter zur Aufnahme von Körperflüssigkeiten aufweisen, sind insbesondere in der DE 10 2009 038 130 A1 und DE 10 2009 038 131 A1 der Anmelderin vorbeschrieben. Eine derartige Vorrichtung weist einen Anschluss für eine wie hier in Rede stehende zum Körper eines Patienten führende Saugleitung auf. Die Merkmale dieser vorbekannten Vorrichtungen seien durch Bezugnahme auf die vorgenannten Anmeldungen auch in diese Anmeldung einbezogen. Die erfindungsgemäß vorgesehene Fluidzuführleitung und eine hiermit zusammenwirkende Fluidzuführvorrichtung kann entweder losgelöst und separat von einer Vorrichtung zur Bereitstellung von Unterdruck für medizinische Anwendungen verwirklicht werden, etwa in Form einer Instillationseinrichtung, oder die Fluidzuführvorrichtung kann in die genannte Vorrichtung zur Bereitstellung von Unterdruck integriert sein. Durch die weiteren Merkmale der Wundauflage mit Hülse kann eine einfache und sichere Applikation der in den Wundraum ragenden Fluidzuführleitung realisiert werden. Die Fluidzuführleitung kann damit innerhalb des Wundraums orientiert werden, und es kann sichergestellt werden, dass zuzuführende Flüssigkeiten bis zum Wundgrund gelangen, wo sie typischerweise benötigt werden. Insbesondere wird durch die vorstehend erwähnte Hülse verhindert, dass eingeleitete Flüssigkeit gleich wieder an die Wundauflage abgegeben und abgesaugt wird. Dadurch, dass die Fluidzuführleitung in die Wundauflage und in die darin vorgesehene Hülse einragt, wird ein unmittelbarer Kontakt der Fluidzuführleitung mit dem Wundgrund und damit eine Reizung des Wundgrunds sicher vermieden.

Nach einer bevorzugten Ausbildung der erfindungsgemäßen Anschlussvorrichtung erweist es sich als vorteilhaft, wenn die Fluidzuführleitung zumindest im Bereich der Anschlussvorrichtung parallel zu der Saugleitung geführt ist. Dies vereinfacht die Anbringung auf dem Unterdruckverband, aber auch die Anordnung und Führung der Leitungsmittel gegenüber dem Patienten. Dessen ungeachtet erweist sich eine parallele Führung der Leitungsmittel im Bereich des Trägermittels als zweckmäßig.

Es sei an dieser Stelle darauf hingewiesen, dass das flächenhafte unterdruckdichte und biegsame Trägermittel für die Saugleitung und in der Folge auch für die Fluidzuführleitung eine flächenhaft ausgebildete Schnittstelle zwischen der Saugleitung und dem Unterdruckverband bezeichnet. Das Trägermittel bildet zusammen mit der Saugleitung und der weiter herangeführten Fluidzuführleitung einen flachen, biegsamen und flächenhaft anmutenden Aufbau. Das Trägermittel ist jedenfalls in der Draufsicht flächenhaft ausladender ausgebildet als die daran anschließende Saugleitung und die zusätzlich herangeführte Fluidzuführleitung. Somit bildet das Trägermittel einen größeren Anschlussbereich gegen die wundabgewandte Oberseite des Unterdruckverbands.

Des weiteren erweist es sich als vorteilhaft, wenn die Saugleitung und die Fluidzuführleitung aus einem biegsamen elastomeren Material einer Shore A-Härte von höchstens 65, insbesondere von 5-65, insbesondere von 10-60, insbesondere von 15-55 gebildet sind. Die Shore -A Härte wird nach DIN 53505 vom August 2000 bestimmt, und zwar bei 23°C an einem wie in der Norm beschrieben plattenförmigen ebenen und glatten Probekörper einer Dicke von 6 mm. In entsprechender Weise erweist es sich als vorteilhaft, wenn auch das flächenhafte Trägermittel aus einem Material des vorgenannten Shore A-Härtebereichs gebildet ist.

Weiter erweist es sich als vorteilhaft, wenn die Fluidzuführleitung in einem spitzen Winkel von insbesondere 10-70°, insbesondere von 20-70° aus der wundzugewandten Seite des Trägermittels austritt. Aufgrund der vorzugsweise biegsamen Ausbildung der Fluidzuführleitung, lässt sich der von der Unterseite des Trägermittels vorstehende Abschnitt der Fluidzuführleitung leicht in die erforderliche gewünschte Position relativ zu dem Wundraum oder der dort vorgesehenen Wundauflage bringen.

Nach einer weiteren Ausführungsform der Erfindung kann es sich für bestimmte Anwendungen als vorteilhaft erweisen, wenn die Fluidzuführleitung an einem von der wundzugewandten Seite des Trägermittels vorstehenden Endabschnitt Queröffnungen in der Wandung aufweist.

Obschon die Länge des von der wundzugewandten Seite des Trägermittels vorstehenden Abschnitts herstellerseitig an sich keinen Restriktionen unterliegt, erweist es sich für die meisten Anwendungen als vorteilhaft, wenn diese Länge des vorstehenden Abschnitts der Fluidzuführleitung 0,5 - 10 cm, insbesondere 0,7 - 5 cm, insbesondere 1 - 3 cm beträgt, wobei dies in einer Längsmittelachse der Fluidzuführleitung gemessen wird. Es wäre insbesondere denkbar, dass herstellerseitig eine verhältnismäßig große Länge vorgesehen wird, die dann unmittelbar beim Anbringen der Anschlussvorrichtung auf das gewünschte Maß gekürzt wird.

Weiter kann die Fluidzuführleitung einlumig oder mehrlumig ausgebildet sein, je nach dem, ob die Zuführung verschiedener Fluids wünschenswert erscheint.

In Weiterbildung der Erfindung wird vorgeschlagen, dass die Fluidzuführleitung im Bereich zwischen der Anschlussvorrichtung und einer Unterdruck erzeugenden Vorrichtung eine Abklemmeinrichtung aufweist, die vorzugsweise manuell betätigbar ist und insbesondere von einem einfachen Klemmmittel, wie einer Klemmschere oder dergleichen gebildet sein kann. Auf diese Weise kann bei einem Verbandwechsel oder beim Anschluss oder Lösen an eine Fluidzuführvorrichtung ein Austropfen von Fluid verhindert werden.

Es kann sich weiter als vorteilhaft erweisen, wenn parallel zu der Saugleitung eine zusätzliche Belüftungsleitung vorgesehen ist und die beiden Leitungen an ihrem jeweiligen Ende im Bereich des Trägermittels miteinander kommunizieren. Eine solche Belüftungsleitung dient einerseits dem Auflösen von Verstopfungen in der Saugleitung, in dem durch Belüften mittels eines Gases, vorzugsweise Luft, ein hinreichend großer Medienstrom zur Verfügung gestellt wird, der im Zusammenwirken mit der Unterdruck erzeugenden Einrichtung eine Verstopfung zu lösen vermag. Auf der anderen Seite vermag eine Belüftungsleitung aber auch zur präzisen Unterdrucksteuerung verwendet werden, wenn nämlich entsprechend eines Steuerprogramms der Unterdruck abgesenkt werden soll (also der absolut herrschende Druck erhöht werden soll). Es wäre auch denkbar, dass die Saugleitung, die Fluidzuführleitung und die Belüftungsleitung in ein gemeinsames Leitungsmittel integriert sind, also insbesondere durch voneinander separate Lumen desselben, insbesondere extrudierten Leitungsmittels gebildet sind, wobei dann vorzugsweise die Saugleitung zwischen den beiden anderen Leitungen ausgebildet ist.

Zum Befestigen der Anschlussvorrichtung an der wundabgewandten Seite eines die Wunde überfangenden Unterdruckverbands sind mehrere Möglichkeiten denkbar: Nach einer Ausführungsform kann die Anschlussvorrichtung mittels einer die Anschlussvorrichtung ganz oder teilweise überfangenden kleberbeschichteten Folienschicht gegen den Unterdruckverband befestigt werden. Diese Folienschicht erstreckt sich dann über die flächenhafte Erstreckung des Trägermittels hinaus. Nach einer weiteren Ausführungsform, kann auf der wundzugewandten Seite des Trägermittels eine Haftvermittlungsschicht vorgesehen sein, die im einfachsten Fall aus einem flächenhaft durchgehenden oder unterbrochenen oder rahmenförmigen Kleberauftrag gebildet sein kann. In Weiterbildung dieser Ausführungsform wird vorgeschlagen, dass auf der wundzugewandten Seite des Trägermittels eine wenigstens dreilagige Haftvermittlungsschicht vorgesehen ist, die eine mittlere Traglage, eine an der Traglage gehaltene und dem Trägermittel zugewandte erste Kleberschicht und eine an der Traglage gehaltene und von dem Trägermittel abgewandte zweite Kleberschicht umfasst und die so ausgebildet ist, dass sie die wenigstens eine Öffnung in dem Trägermittel nicht blockiert.

Diese erfindungsgemäße Ausbildung der Anschlussvorrichtung erweist sich als besonders zweckmäßig, da die Haftvermittlungsschicht typischerweise in einer idealen ebenen Erstreckung gegen die ebene wundzugewandte Seite des flächenhaften Trägermittels fixiert werden kann, während sonst eine zusätzliche Klebefolie das Trägermittel bzw. den Aufbau des Trägermittels überfangen muss und so eine zusätzliche dritte Dimension hinzukommt, welche bei der praktischen Handhabung aufgrund der Bildung von Falten den ordnungsgemäßen Vorgang des Aufbringens bzw. Anhaftens der Anschlussvorrichtung gegen die wundabgewandte Oberseite des Wundverbands erschwert. Ein weiterer Vorteil dieser Ausbildung ergibt sich aus dem Umstand, dass für das flächenhafte Trägermittel einerseits und den Wundverband andererseits typischerweise verschiedene Materialien, beispielsweise einerseits Silikon und beispielsweise andererseits Polyurethan, verwendet werden, so dass der Kleber einer Klebefolie nicht optimal auf beide Materialien abgestimmt werden kann. Insofern erweist es sich als vorteilhaft, wenn die erste Kleberschicht und die zweite Kleberschicht von unterschiedlichen Klebematerialien mit unterschiedlichen Klebeeigenschaften gebildet sind, so dass sie entsprechend den spezifischen Materialien von Trägermittel bzw. Unterdruckverband ausgewählt werden können.

Insbesondere kann es sich hierbei als vorteilhaft erweisen, wenn das Trägermittel aus Silikon gebildet ist und die erste Kleberschicht einen Silikonkleber, zumindest als Hauptkomponente, umfasst. Weiter kann es sich als vorteilhaft erweisen, wenn die zweite Kleberschicht einen Acrylatkleber, zumindest als Hauptkomponente, umfasst. Acrylatkleber vermitteln eine hervorragende Anhaftung an Polyurethanoberflächen. Solchenfalls kann also eine optimale Anhaftung an einem Wundverband mit einer Polyurethanoberfläche erreicht werden.

Nach einem weiteren Erfindungsgedanken erweist es sich als vorteilhaft, wenn die klebende Fläche der ersten Kleberschicht zu dem Trägermittel hin und/oder die klebende Fläche der zweiten Kleberschicht zu dem Unterdruckverband hin kleiner als 100 cm², insbesondere kleiner als 50 cm², insbesondere kleiner als 40 cm², insbesondere kleiner als 30 cm² ist. Es hat sich gezeigt, dass bei einem optimal auf den Haftpartner, also das Material des Trägermittels bzw. des Wundverbands, abgestimmten Klebermaterial zur Erreichung derselben Haftwirkung eine sehr viel geringere klebende Fläche bei der Anschlussvorrichtung eingesetzt werden muss. Die vorstehenden Flächenangaben beziehen sich hierbei auf die einseitig projizierte Fläche der betreffenden Kontaktseite, also schlicht und einfach auf die Größe der klebenden ebenen Fläche der ersten bzw. zweiten Kleberschicht.

Es erweist sich als besonders vorteilhaft, wenn bei der erfindungsgemäßen Anschlussvorrichtung eine Haftvermittlungsschicht verwendet ist, deren erste Kleberschicht so ausgebildet ist, dass die Haftvermittlungsschicht eine durch diese erste Kleberschicht vermittelte Schälkraft gegenüber einer Stahlplatte von 25 bis 40 N/100 mm (100 mm Breite der ersten Kleberschicht) aufweist und/oder deren zweite Kleberschicht so ausgebildet ist, dass die Haftvermittlungsschicht eine durch diese zweite Kleberschicht vermittelte Schälkraft gegenüber einer Stahlplatte von 70 bis 85 N/100 mm (100 mm Breite der zweiten Kleberschicht) aufweist. Es wird hierbei die Haftvermittlungsschicht als solche, also nicht im Verbund mit dem Trägermittel getestet. Diese Schälkraft wird bestimmt nach ASTM Testmethode D 3330 M, wobei die Verweilzeiten ³ 1 min betragen, der Abzugswinkel beträgt 180° (Peel-off-Orientierung) und die Abzugsgeschwindigkeit beträgt 300 mm/min. Der Test wird bei 22°C und 50 % relativer Luftfeuchte ausgeführt. In vorteilhafter Weise lassen sich diese Werte erreichen, wenn die erste Kleberschicht einen Silikonkleber umfasst bzw. wenn die zweite Kleberschicht einen Acrylatkleber umfasst.

Es erweist sich herstellungstechnisch als zweckmäßig, wenn die erste und die zweite Kleberschicht dieselbe flächenhafte Erstreckung in Projektion auf ihre Erstreckungsebene aufweisen, also in Projektion deckungsgleich sind, und insbesondere dieselbe Erstreckung wie die Traglage aufweisen. Im letzteren Fall sind also alle Lagen deckungsgleich ausgebildet. Diese Ausgestaltung erweist sich als besonders zweckmäßig, weil solchenfalls die Haftvermittlungsschicht ausgehend von einem endlosen zweiseitig mit Klebematerial beschichteten Flachmaterialband hergestellt werden kann, indem die für die Haftvermittlungsschicht erforderlichen Abschnitte lediglich abgelängt oder ausgestanzt zu werden brauchen.

Die Haftvermittlungsschicht kann in besonders vorteilhafter Weise ausgehend von einem doppelseitigen Klebeband hergestellt werden, wobei ein Abschnitt des doppelseitigen Klebebands abgetrennt und entsprechend größenmäßig und formmäßig konfiguriert und sodann auf die wundzugewandte Seite des Trägermittels aufgebracht wird.

Die Haftvermittlungsschicht ist mit ihrer ersten Kleberschicht vorzugsweise unmittelbar auf die wundzugewandte Seite des Trägermittels aufgebracht.

Weiter erweist es sich als vorteilhaft, wenn die erste und die zweite Kleberschicht eine Dicke von 20 bis 400 µm aufweist.

Bei der mittleren Traglage kann es sich in vorteilhafter Weise um ein Vliesmaterial, ein Flachmaterial mit einer textilen Bindung, wie z. B. ein Gestrick, Gewirke oder Gewebe, oder um eine Kunststofffolie, eine Metallfolie oder einen Verbundwerkstoff hieraus handeln.

Es erweist sich weiter als vorteilhaft, wenn sich die Haftvermittlungsschicht über die gesamte wundzugewandte Seite des Trägermittels erstreckt, wobei hierbei die wenigstens eine Öffnung in dem Trägermittel zum Zwecke der Unterdruckkommunikation ausgenommen ist.

Es ist auch denkbar und vorteilhaft, wenn sich die Haftvermittlungsschicht rahmenförmig um die wenigstens eine Öffnung in dem Trägermittel herum erstreckt. Solchenfalls kann die flächenhafte Erstreckung der Haftvermittlungsschicht auch wesentlich geringer sein als die flächenhafte Erstreckung der wundzugewandten Seite des Trägermittels. Es erweist sich jedoch gleichwohl als vorteilhaft, wenn die flächenhafte Erstreckung der Haftvermittlungsschicht wenigstens 50 % der Fläche der wundzugewandten Seite des Trägermittels beträgt.

Weiter erweist es sich als vorteilhaft, wenn die zweite Kleberschicht von einer ablösbaren Schutzschicht überfangen ist, die vorzugsweise zweiteilig und vorzugsweise mit einer Anfasslasche und/oder einem über die zweite Kleberschicht überstehenden und ergreifbaren Bereich ausgebildet ist.

Die Breitenerstreckung der flachen Saugleitung beträgt vorzugsweise wenigstens 10 mm, insbesondere wenigstens 15 mm und weiter insbesondere wenigstens 18 mm und insbesondere höchstens 30 mm und weiter insbesondere höchstens 25 mm. Nach einer bevorzugten Ausführungsform der Erfindung ist die Saugleitung auf Silikonbasis ausgebildet.

Weiter erweist es sich als vorteilhaft, wenn die Saugleitung im Inneren angeformte, insbesondere mit dem Material der Saugleitung einstückig ausgebildete Mittel zur Verhinderung des Kollabierens der Saugleitung bei Unterdruckbeaufschlagung aufweist. Diese Mittel zur Verhinderung des Kollabierens können beispielsweise von Rippen oder Vorsprüngen gebildet sein. In Weiterbildung dieses Erfindungsgedankens erweist es sich als vorteilhaft, wenn diese durchgehend erstreckt sind. Die Saugleitung kann dann in vorteilhafter Weise als Extrusionsteil ausgebildet sein.

Es kann sich ferner als vorteilhaft erwiesen, wenn die Saugleitung mehrere voneinander druckdicht abgetrennte Kanäle umfasst, wobei die Saugleitung auch solchenfalls vorzugsweise einstückig ausgebildet ist, also keine Zusammenfassung von mehreren separaten kanalbildenden Mitteln aufweist. Einer der mehreren Kanäle kann dabei die Fluidzuführleitung bilden. Ein anderer die Belüftungsleitung.

Vorzugsweise erstreckt sich die vorzugsweise flache Saugleitung über eine gewisse Distanz in Längsrichtung und kann dann über ein nicht dargestelltes Übergangs- oder Kopplungselement, welches eine Steckverbindung oder Klebeverbindung bilden kann, in einen üblichen verwindungssteiferen Rundschlauch übergehen, der zu einer Unterdruck erzeugenden Vorrichtung führt, die als stationäres Gerät oder als mobil am Körper des Patienten tragbares Gerät ausgebildet sein kann. Das Übergangs- oder Kopplungselement kann auch zum Koppeln eines mehrkanaligen Leitungsmittels mit einem mehrkanaligen Rundschlauch ausgebildet sein. Als zweckmäßige Längserstreckung der Saugleitung hat sich eine Strecke von 10 - 60 cm erwiesen.

Das flächenhafte Trägermittel weist vorteilhafterweise eine Dicke von 0,75 - 3 mm, insbesondere von 1 - 3 mm auf. Es ist nach einer bevorzugten Ausführungsform der Erfindung auf Silikonbasis ausgebildet. Nach einer weiteren bevorzugten Ausführungsform ist das Saugleitung und das Trägermittel aus demselben elastomeren Material ausgebildet.

Es erweist sich als vorteilhaft, wenn die flächenhafte Erstreckung des Trägermittels wenigstens das 1,5-fache vorzugsweise wenigstens das Doppelte der senkrecht auf das Trägermittel projizierten Fläche der Saugleitung umfasst, da hierdurch die über die Saugleitung bei Berührung eingeleiteten Kräfte über eine größere Fläche verteilt werden und auch Biegemomente, die auf die Saugleitung ausgeübt werden, nicht oder in geringerem Maße auf den Unterdruckverband übertragen werden; sie werden besser durch das plattenförmige Trägermittel aufgenommen. Es erweist sich als ausreichend, wenn das vorstehend erwähnte Flächenverhältnis höchstens 5, insbesondere höchstens 4 beträgt, wobei sich ein Verhältnis von 2 - 3 als vorteilhaft erwiesen hat.

Der unterdruckdichten Verbindung der flachen Saugleitung mit der wundabgewandten Oberseite des Trägermittels kommt eine funktional wesentliche Bedeutung zu. Es kommt hierfür grundsätzlich eine Klebeverbindung unter Verwendung eines Haftvermittlers im weitesten Sinn in Frage. Weiter erweist sich eine thermische Fügeverbindung als vorteilhaft, die einer Art Vulkanisierverbindung entsprechen kann. Beispielsweise kann das zuvor separat hergestellte Leitungsmittel auf das gerade frisch ausgegossene und nur teilweise erstarrte flächenhafte Trägermittel aufgebracht werden, so dass hierbei eine stoffschlüssige innige Anbindung der beiden Komponenten ohne Verwendung eines zusätzlichen Haftvermittlers erzielt wird.

Es erweist sich als vorteilhaft, wenn die miteinander kommunizierenden Öffnungen in der Saugleitung und in dem Trägermittel einander überdecken, also miteinander fluchten. Dies erweist sich als am einfachsten herstellbar, wenn diese Öffnungen erst nach dem unterdruckdichten Verbinden des flachen Leitungsmittels mit dem flächenhaften Trägermittel gleichzeitig in beiden Komponenten ausgebildet werden. Dies kann beispielsweise durch einen materialherausformenden Stanzvorgang erreicht werden.

Im Hinblick auf die Anzahl und die Größe der Öffnungen in der Saugleitung und in dem flächenhaften Trägermittel wäre es an sich denkbar, dass nur eine einzige Öffnung vorgesehen ist. Es erweist sich jedoch als vorteilhaft, wenn im unterdruckdichten Verbindungsbereich von Saugleitung und Trägermittel mehrere Öffnungen, insbesondere wenigstens zwei, insbesondere wenigstens vier Öffnungen je cm Länge der Saugleitung vorgesehen sind.

Es erweist sich auch als vorteilhaft, wenn die lichte Öffnungsfläche der Öffnungen 5 - 50 % der Fläche der miteinander unlösbar gefügten Flachseiten der Saugleitung und des Trägermittels beträgt.

Weitere Merkmale, Einzelheiten und Vorteile der Erfindung ergeben sich aus den beigefügten Patentansprüchen und aus der nachfolgenden Beschreibung einer bevorzugten Ausführungsform der erfindungsgemäßen Anschlussvorrichtung und ihrer Anwendung bei der Unterdruckbehandlung von Wunden.

In der Zeichnung zeigt:
Figur 1 eine perspektivische nicht maßstabsgetreue Ansicht einer erfindungsgemäßen Anschlussvorrichtung zur Verwendung bei der Unterdruckbehandlung von Wunden, sowie eine angedeutete Wunde mit einem Unterdruckwundverband; und
Figur 2 eine schematische nicht maßstabsgetreue Schnittansicht der Anschlussvorrichtung nach Figur 1 mit Schnittebene II-II;
Figur 3 eine schematische nicht maßstabsgetreue Schnittansicht der Anschlussvorrichtung nach Figur 1 mit Schnittebene III-III;
Figur 4 eine schematische nicht maßstabsgetreue Schnittansicht der Anschlussvorrichtung im Zusammenwirken mit einer Wundauflage.

Die Figuren zeigen verschiedene Ansichten einer erfindungsgemäßen insgesamt mit dem Bezugszeichen 2 bezeichneten Anschlussvorrichtung zur Verwendung bei der Unterdruckbehandlung von Wunden. Die dargestellte Anschlussvorrichtung 2 wird auf eine wundabgewandte Oberseite 4 eines schematisch Unterdruckverbands 6, welcher eine zu behandelnde Wunde 8 überfängt und zur Atmosphäre hin unterdruckdicht abschließt, aufgebracht und daran auf noch zu beschreibende weise klebend befestigt.

Die Anschlussvorrichtung 2 umfasst ein beispielhaft dargestelltes flaches Leitungsmittel in Form einer Saugleitung 10 aus einem elastomeren biegsamen Material und ein flächenhaft erstrecktes, plattenförmiges Trägermittel 12, welches die flache Saugleitung 10 hält und stützt, so dass auf die Saugleitung 10 ausgeübte Druck- oder Biegekräfte gleichmäßig in das flächenhafte Trägermittel 12 eingeleitet und von diesem aufgenommen werden können.

Die Saugleitung 10 ist derart flachbauend ausgebildet, dass sie in einem Längsendabschnitt 14 mit nahezu 100 % ihrer senkrecht auf das Trägermittel 12 projizierten Fläche mit dem Trägermittel 12 verbunden ist. Die Saugleitung 10 kann mit ihrer betreffenden Flachseite 15 auf die wundabgewandte Oberseite 16 des Trägermittels 12 aufgeklebt oder aufvulkanisiert, also thermisch gefügt sein.

Vorzugsweise nach dem unterdruckdichten Fügen der Saugleitung 10 mit dem Trägermittel 12 werden durch das Trägermittel 12 und durch die Saugleitung 10 hindurchgehende Öffnungen 18 ausgebildet, die mit einer schematisch dargestellten Öffnung 20 oder mehreren Öffnungen 20 in dem Unterdruckverband 6 kommunizieren, wenn die Anschlussvorrichtung 2 auf den Unterdruckverband 6 appliziert ist, um einen Unterdruck in den Wundraum anzulegen.

Die Dickenerstreckung D des Verbunds aus Saugleitung 10 und Trägermittel 12 beträgt höchstens 7 mm, vorzugsweise höchstens 5 mm und weiter vorzugsweise lediglich 3 - 4 mm.

Aus den Figuren erkennt man weiter, dass die Anschlussvorrichtung 2 auf der wundzugewandten Seite 22 des Trägermittels 12 eine Haftvermittlungsschicht 24 aufweist. Mittels dieser Haftvermittlungsschicht 24 ist die Anschlussvorrichtung 2 gegen die wundabgewandte Oberseite 4 des Unterdruckverbands 6 klebend haftend aufbringbar. Die Haftvermittlungsschicht 24 ist dreilagig ausgebildet und umfasst eine mittlere Traglage 26, eine an der Traglage 26 gehaltene und dem Trägermittel 12 zugewandte erste Kleberschicht 28 und eine an der Traglage 26 gehaltene und von dem Trägermittel 12 abgewandte, d. h. dem Unterdruckverband 6 zugewandte zweite Kleberschicht 30. Im beispielhaft und bevorzugt dargestellten Fall sind alle drei Lagen der Haftvermittlungsschicht 24 deckungsgleich miteinander ausgebildet. Die Haftvermittlungsschicht 24 ist als Ganzes von einem beidseitig mit Klebermaterial beschichteten Flachmaterial abgelängt bzw. ausgestanzt. Sie ist im beispielhaft dargestellten Fall rahmenförmig, also in Umfangsrichtung durchgehend ausgebildet und erstreckt sich um denjenigen zentralen Bereich 32 des Trägermittels 12 herum, in dem die schematisch dargestellten Öffnungen 18 des Trägermittels 12 bzw. der Saugleitung 10 in Richtung auf den Unterdruckverband 6 ausmünden. Die klebende Fläche der ersten und zweiten Kleberschichten 28, 30 beträgt im beispielhaft dargestellten Fall nur ca. 30 cm². Es sei an dieser Stelle aber auch ausdrücklich erwähnt, dass sich die Haftvermittlungsschicht 24 auch über die gesamte wundzugewandte Seite 22 des Trägermittels 12 erstrecken kann, wobei sie solchermaßen mit den Öffnungen 18 in dem Trägermittel 12 bzw. in der Saugleitung 10 fluchtende oder kommunizierende Durchtrittsöffnungen aufweisen muss. Die klebende Fläche beträgt dann gleichwohl nur ca. 40 cm².

Die Kleberschichten 28 und 30 sind von unterschiedlichen Klebematerialien gebildet, die entsprechend auf die Materialien des Trägermittels 12 bzw. des Unterdruckverbands 6 optimiert sind. Sofern beispielsweise das Trägermittel 12 aus Silikon gebildet ist, empfiehlt es sich, dass die erste Kleberschicht 28 einen Silikonkleber umfasst. Wenn derjenige Unterdruckverband 6, für den die Anschlussvorrichtung 2 konzipiert ist, aus Polyurethan bzw. mit einer wundabgewandten Oberseite 4 aus Polyurethan ausgebildet ist, so empfiehlt es sich, dass die zweite Kleberschicht 30 einen Acrylatkleber umfasst, der besonders gut auf Polyurethan haftet. Durch die Verwendung der erfindungsgemäßen Haftvermittlungsschicht 24 können jedenfalls deren beide Kleberschichten 28 und 30 optimal auf die mit ihnen haftend zusammenwirkenden Materialien des Trägermittels 12 bzw. des Unterdruckverbands 6 abgestimmt und herstellerseitig in ihrer Zusammensetzung ausgebildet werden.

Die zweite Kleberschicht 30 ist zudem von einer zweiteiligen ablösbaren Schutzschicht 34 überfangen, die eine Anfasslasche 36 zum Ablösen der beiden Teile aufweist.

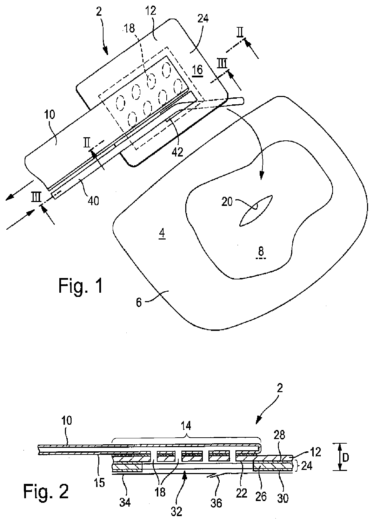

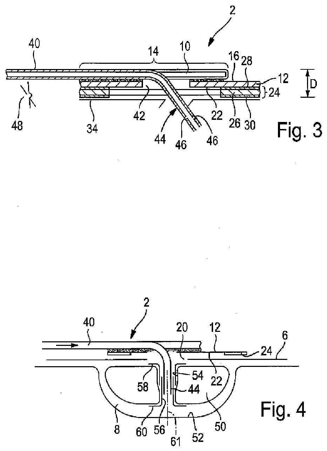

Man erkennt in den Figuren 1 und 3 des weiteren eine Fluidzuführleitung 40, mit der ein Fluid, beispielsweise zur Wundspülung oder zur Instillation der Wunde dem Wundraum zugeführt werden kann. Diese Fluidzuführleitung 40 ist vorzugsweise in derselben Ebene wie die Saugleitung 10 und parallel zu der Saugleitung 10 an das Trägermittel 12 herangeführt; sie ist ebenfalls von dem Trägermittel 12 gehalten und an die wundabgewandte Oberseite 16 des Trägermittels 12, beispielsweise mittels Kleber oder stoffschlüssig unlösbar angefügt. Erfindungsgemäß erstreckt sich die Fluidzuführleitung 40 durch eine weitere Öffnung 42 in dem Trägermittel 12 hindurch auf die wundzugewandte Seite 22 des Trägermittels 12 und steht dort in einem spitzen Winkel in Richtung auf die Wunde vor. Da die Fluidzuführleitung 40 aus einem biegsamen Material der eingangs beschriebenen Art gebildet ist, lässt sich ein vorstehender Abschnitt 44 der Fluidzuführleitung 40 leicht und im wesentlichen widerstandslos in andere gewünschte Richtungen abbiegen und auf noch zu beschreibende Weise bezüglich der Wunde bzw. einer Wundauflage orientieren. In dem ausragenden Abschnitt 44 der Fluidzuführleitung 40 können für bestimmte Anwendungen zusätzliche Queröffnungen 46 in der Wandung ausgebildet sein, was lediglich schematisch angedeutet ist. Des Weiteren ist schematisch eine Abklemmeinrichtung 48 angedeutet, mittels der der Querschnitt der Fluidzuführleitung 40 auf Null reduziert werden kann.

Figur 4 zeigt stark schematisiert die Anwendung der erfindungsgemäßen Anschlussvorrichtung 2 zusammen mit einer Wundauflage 50 bei einer Wunde 8, die mit einem Unterdruckverband 6 bis auf die Öffnung 20 abgedichtet ist. Im Bereich dieser Öffnung 20 in dem Unterdruckverband 6 ist die Anschlussvorrichtung 2 insbesondere mittels der erwähnten Haftvermittlungsschicht 24 dichtend aufgebracht. Alternativ könnte die Anschlussvorrichtung 2 mittels zusätzlicher die Anschlussvorrichtung 2 überfangender Klebefolien oder dergleichen dichtend an der wundabgewandten Oberseite 16 des Trägermittels 12 fixiert werden. In der schematisierten Schnittdarstellung der Figur 4 ist nur die Schnittebene durch die Fluidzuführleitung 40 erfasst. Man erkennt, dass der von der wundzugewandten Seite 22 des Trägermittels 12 vorstehende Abschnitt 44 der Fluidzuführleitung 40 in die Wundauflage 50 in Richtung auf den Wundgrund 52 eingeführt ist. Hierfür ist in der Wundauflage 50 erfindungsgemäß eine Durchgangsöffnung 54 ausgebildet. In der Durchgangsöffnung 54 ist eine Hülse 56 angeordnet. Die Hülse 56 ist zweiteilig in Form zweier von jeweils gegenüberliegenden Seiten der Wundauflage 50 in die Durchgangsöffnung 54 eingeführter Hülsenteile 58, 60 ausgebildet. Die Hülsenteile 58, 60 sind zueinander teleskopierend entlang einer Hülsenlängsachse 61. Auf diese Weise lässt sich die Hülsenlänge entsprechend der Deformation der Wundauflage 50 selbsttätig einstellen. Je nach Unterdruck und Komprimierung der vorzugsweise aus einem absorbierenden Schaum gebildeten Wundauflage 50 kann die Hülsenlänge selbsttätig eingestellt werden. Die Hülse 58 führt den Abschnitt 44 der Fluidzuführleitung 40 und verhindert, dass dieser gegen den Wundgrund 52 anliegt. Auf diese Weise kann Flüssigkeit über die Fluidzuführleitung 40 an tief gelegene Bereiche der Wunde 8, vorzugsweise bis zum Wundgrund 52 zugeführt werden. Zugeführte Flüssigkeit kann dann zusammen mit Wundflüssigkeiten und Wundsekreten über die in Figur 4 nicht dargestellte Saugleitung 10 abgeführt werden.

Die Anordnung und Ausbildung der Hülse 56 ist rein beispielhaft. Die Erfindung ist durch die folgenden Ansprüche definiert.

## Patentansprüche

1. Sachgesamtheit zur Verwendung bei der Unterdruckbehandlung von Wunden aus einer Anschlussvorrichtung (2), einem Unterdruckverband (6) und einer, insbesondere ein Schaummaterial aufweisenden Wundauflage (50) auf der wundzugewandten Seite des Unterdruckverbands (6), wobei die Anschlussvorrichtung (2) mit einer mit Unterdruck beaufschlagbaren Saugleitung (10) und mit einem flächenhaften unterdruckdichten und biegsamen Trägermittel (12) für die Saugleitung (10) ausgebildet ist, an dem die Saugleitung (10) unterdruckdicht gehalten ist, wobei das Trägermittel (12) auf den die Wunde überfangenden und gegen die Atmosphäre dichtend abschließenden Unterdruckverband (6) im wesentlichen unterdruckdicht aufbringbar ist, so dass die Saugleitung (10) durch wenigstens eine Öffnung (18) in dem Trägermittel (12) und in dem Unterdruckverband (6) hindurch mit dem Wundraum kommunizieren kann, wobei die Saugleitung (10) biegsam und flach ausgebildet ist und in einem wundseitigen Längsabschnitt (8) mit wenigstens 70 % ihrer senkrecht auf das Trägermittel (12) projizierten Fläche mit dem Trägermittel (12) für den bestimmungsgemäßen Gebrauch unlösbar und flächenhaft verbunden ist, wobei das Trägermittel (12) und die Saugleitung (10) mit ihren aus ihrer flachen Form resultierenden Hauptebenen zueinander parallel sind, und wobei die Dickenerstreckung (D) des Verbunds aus Saugleitung (10) und Trägermittel (12) höchstens 7 mm beträgt, **dadurch gekennzeichnet, dass** eine in derselben Ebene wie die Saugleitung (10) an das Trägermittel (12) herangeführte Fluidzuführleitung (40) zur Zufuhr eines fluiden Mediums zum Wundraum vorgesehen ist, und dass deren Dicke nicht größer als die Dicke der Saugleitung (10) ist, und dass die Fluidzuführleitung (40) durch eine Öffnung (42) in dem Trägermittel (12) dichtend hindurchgeführt ist und von der wundzugewandten Seite (22) des Trägermittels (12) vorsteht und durch die Öffnung (20) in dem Unterdruckverband (6) hindurch in den Wundraum und in die dort vorgesehene Wundauflage (50) einragen kann, und dass die Wundauflage (50) hierfür eine in Wundtiefenrichtung erstreckte Durchgangsöffnung (54) aufweist und dass die Durchgangsöffnung (54) in der Wundauflage (50) von einer Einsteckhülse (56) begrenzt ist, in welche die Fluidzuführleitung (40) mit ihrem von der wundzugewandten Seite (22) des Trägermittels (12) vorstehenden Abschnitt (44) einführbar ist.

2. Sachgesamtheit nach Anspruch 1, **dadurch gekennzeichnet, dass** die Fluidzuführleitung (40) in einem spitzen Winkel, von insbesondere 30-70°, aus der wundzugewandten Seite (22) des Trägermittels (12) austritt.

3. Sachgesamtheit nach Anspruch 1 oder 2, **dadurch gekennzeichnet, dass** die Fluidzuführleitung (40) an einem von der wundzugewandten Seite (22) des Trägermittels (12) vorstehenden Endabschnitt Queröffnungen (46) in der Wandung aufweist.

4. Sachgesamtheit nach einem oder mehreren der vorstehenden Ansprüche, **dadurch gekennzeichnet, dass** die Länge des von der wundzugewandten Seite (22) des Trägermittels (12) vorstehenden Abschnitts der Fluidzuführleitung (40) 0,5 - 10 cm, insbesondere 0,7 - 5 cm, insbesondere 1 - 3 cm beträgt.

5. Sachgesamtheit nach einem oder mehreren der vorstehenden Ansprüche, **dadurch gekennzeichnet, dass** auf der wundzugewandten Seite (22) des Trägermittels (12) eine wenigstens dreilagige Haftvermittlungsschicht (24) vorgesehen ist, die eine mittlere Traglage (26), eine an der Traglage (26) gehaltene und dem Trägermittel (12) zugewandte erste Kleberschicht (28) und eine an der Traglage (26) gehaltene und von dem Trägermittel (12) abgewandte zweite Kleberschicht (30) umfasst und die so ausgebildet ist, dass sie die wenigstens eine Öffnung (18) in dem Trägermittel (12) nicht blockiert.

6. Sachgesamtheit nach Anspruch 5, **dadurch gekennzeichnet, dass** die erste Kleberschicht (28) und die zweite Kleberschicht (30) von unterschiedlichen Klebematerialien mit unterschiedlichen Klebeeigenschaften gebildet sind.

7. Sachgesamtheit nach Anspruch 5 oder 6, **dadurch gekennzeichnet, dass** das Trägermittel (12) aus Silikon gebildet ist und die erste Kleberschicht (28) einen Silikonkleber umfasst.

8. Sachgesamtheit nach Anspruch 6 oder 7, **dadurch gekennzeichnet, dass** die zweite Kleberschicht (30) einen Acrylatkleber umfasst.

9. Sachgesamtheit nach einem oder mehreren der vorstehenden Ansprüche 5-8, **dadurch gekennzeichnet, dass** die klebende Fläche der ersten Kleberschicht (28) zu dem Trägermittel (12) hin und/oder die klebende Fläche der zweiten Kleberschicht (30) zu dem Unterdruckverband (6) hin kleiner als 100 cm², insbesondere kleiner als 50 cm², insbesondere kleiner als 40cm², insbesondere kleiner als 30 cm² ist.

10. Sachgesamtheit nach einem oder mehreren der vorstehenden Ansprüche 5-9, **dadurch gekennzeichnet, dass** eine Haftvermittlungsschicht (24) verwendet ist, deren erste Kleberschicht (28) so ausgebildet ist, dass die Haftvermittlungsschicht (24) eine durch diese erste Kleberschicht vermittelte Schälkraft gegenüber einer Stahlplatte von 25 bis 40 N/100 mm, insbesondere von 27 - 35 N/100 mm, aufweist und/oder deren zweite Kleberschicht (30) so ausgebildet ist, dass die Haftvermittlungsschicht (24) eine durch diese zweite Kleberschicht vermittelte Schälkraft gegenüber einer Stahlplatte von 70 bis 85 N/100 mm, insbesondere von 70 - 80 N/100 mm, aufweist.

11. Sachgesamtheit nach einem oder mehreren der vorstehenden Ansprüche 5-10, **dadurch gekennzeichnet, dass** die erste und die zweite Kleberschicht (28, 30) dieselbe flächenhafte Erstreckung in Projektion auf ihre Erstreckungsebene aufweisen, und insbesondere dieselbe Erstreckung wie die Traglage (26) aufweisen.

12. Sachgesamtheit nach einem oder mehreren der vorstehenden Ansprüche 5-11, **dadurch gekennzeichnet, dass** die erste und die zweite Kleberschicht (28, 30) eine Dicke von 20 - 400 µm aufweisen.

13. Sachgesamtheit nach einem oder mehreren der vorstehenden Ansprüche 5-12, **dadurch gekennzeichnet, dass** die mittlere Traglage (26) ein Vliesmaterial, ein Flachmaterial mit einer textilen Bindung, wie z. B. ein Gestrick, Gewirke oder Gewebe, oder eine Kunststofffolie, eine Metallfolie oder einen Verbundwerkstoff hieraus umfasst oder hieraus gebildet ist.

14. Sachgesamtheit nach einem oder mehreren der vorstehenden Ansprüche 5-13, **dadurch gekennzeichnet, dass** sich die Haftvermittlungsschicht (24) über die gesamte wundzugewandte Seite (22) des Trägermittels (12) erstreckt.

15. Sachgesamtheit nach einem oder mehreren der vorstehenden Ansprüche 5-14, **dadurch gekennzeichnet, dass** die flächenhafte Erstreckung der Haftvermittlungsschicht (24) wenigstens 50 % der Fläche der wundzugewandten Seite (22) des Trägermittels (12) beträgt.

## Claims

1. Aggregate for use in the vacuum therapy of wounds comprised of a connecting device (2), a vacuum dressing (6) and a wound support (50), in particular, having a foam material, on the wound-facing side of the vacuum dressing (6), wherein the connecting device (2) is formed with a suction tube (10) to which a vacuum can be applied and a two-dimensional vacuum-tight and flexible support means (12) for the suction tube (10), on which the suction tube (10) is held in a vacuum-tight manner, wherein the support means (12) can be applied in an essentially vacuum-tight manner to the vacuum dressing (6) that overlaps the wound and seals it with respect to the atmosphere, so that the suction tube (10) can communicate with the wound space through at least one opening in the support means (12) and in the vacuum dressing (6), wherein the suction tube (10) is constituted to be flexible and flat and is, in its intended use, non-detachably and two-dimensionally connected to the support means (12) in a wound-side longitudinal section (8) with at least 70 % of its surface projected perpendicularly onto the support means (12), wherein the support means (12) and the suction tube (10) are mutually parallel with their main planes resulting from their flat shape, and wherein the thickness (D) of the assembly comprising the suction tube (10) and support means (12) is no more than 7 mm, **characterized in that** a fluid feed tube (40) for conveying a fluid medium to the wound space, which is located in the same plane as the suction tube (10) and which extends to the support means (12) is provided, whose thickness is no greater than the thickness of the suction tube (10), and that the fluid feed tube (40) extends sealingly through an opening (42) in the support means (12) and protrudes beyond the wound-facing side (22) of the support means (12) and which can project through the opening (20) in the vacuum dressing (6) into the wound space or into a wound support (50) provided there and that for that purpose the wound support (50) has a through-hole (54) that extends in the direction of the wound depth, and that the through-hole (54) in the wound support (50) is delimited by an insert sleeve (56) into which the fluid feed tube (40) can be inserted with its section (44) protruding from the wound-facing side (22) of the support means (12).

2. Aggregate according to claim 1, **characterized in that** the fluid feed tube (40) exits at an acute angle, in particular, of 30-70°, from the wound-facing side (22) of the support means (12).

3. Aggregate according to claims 1 or 2, **characterized in that** the fluid feed tube (40) has transverse openings (46) in the wall on an end section protruding from the wound-facing side (22) of the support means (12).

4. Aggregate according to one or more of the previous claims, **characterized in that** the length of the section of the fluid feed tube (40) protruding from the wound-facing side (22) of the support means (12) is 0.5 - 10 cm, in particular, 0.7 - 5 cm, in particular, 1 - 3 cm.

5. Aggregate according to one or more of the previous claims, **characterized in that** on the wound-facing side (22) of the support means (12) an at least three-layer adhesion layer (24) is provided, which comprises a middle support layer (26), a first adhesive layer (28) held on the support layer (26) and facing the support means (12), and a second adhesive layer (30) held on the support layer (26) and facing away from the support means (12), and is constituted such that it does not block at least one opening (18) in the support means (12).

6. Aggregate according to the claim 5, **characterized in that** the first adhesive layer (28) and the second adhesive layer (30) are made from different adhesive materials with different adhesive properties.

7. Aggregate according to either one of the claims 5 or 6, **characterized in that** the support means (12) is made of silicone and the first adhesive layer (28) comprises a silicone adhesive.

8. Aggregate according to either one of the claims 6 or 7, **characterized in that** the second adhesive layer (30) comprises an acrylate adhesive.

9. Aggregate according to one or more of the previous claims 5-8, **characterized in that** the adhesive surface of the first adhesive layer (28) facing the support means (12) and/or the adhesive surface of the second adhesive layer (30) facing the vacuum dressing (6) is smaller than 100 cm², in particular, smaller than 50 cm², in particular, smaller than 40 cm², in particular, smaller than 30 cm².

10. Aggregate according to one or more of the previous claims 5-9, **characterized in that** an adhesion layer (24) is used whose first adhesive layer (28) is constituted such that the adhesion layer (24) exhibits a peel adhesion provided by this first adhesive layer with respect to a steel plate of 25 to 40 N/100 mm, in particular, of 27 - 35 N/100 mm, and/or whose second adhesive layer (30) is constituted such that the adhesion layer (24) has a peel adhesion with respect to a steel plate provided by this second adhesive layer of 70 to 85 N/100 mm, in particular, of 70 - 80 N/100 mm.

11. Aggregate according to one or more of the previous claims 5-10, **characterized in that** the first and the second adhesive layer (28, 30) exhibit the same two-dimensional extension when projected onto their extension plane and, in particular, the same extension as the support layer (26).

12. Aggregate device according to one or more of the previous claims 5-11, **characterized in that** the first and the second adhesive layer (28, 30) have a thickness of 20 - 400 µm.

13. Aggregate according to one or more of the previous claims 5-12, **characterized in that** the middle support layer (26) comprises or is made from a non-woven material, a flat material with textile binding, such as knitted or woven material, or plastic film, a metal film, or a composite material.

14. Aggregate according to one or more of the previous claims 5-13, **characterized in that** the adhesion layer (24) extends across the entire wound-facing side (22) of the support means (12).

15. Aggregate according to one or more of the previous claims 5-14, **characterized in that** the two-dimensional extension of the adhesion layer (24) is at least 50 % of the surface of the wound-facing side (22) of the support means (12).

## Revendications

1. Ensemble matériel destiné à être utilisé dans le traitement de plaies par pression négative, se composant d'un dispositif de raccordement (2), d'un pansement à pression négative (6) ainsi que, sur la face du pansement à pression négative (6) qui montre vers la plaie, d'un pansement pour plaie (50) présentant en particulier une matière de mousse, dans lequel ledit dispositif de raccordement (2) est réalisé avec une conduite d'aspiration (10) qui peut être mise en pression négative et avec un moyen support (12) en nappe pour la conduite d'aspiration (10), qui est étanche à la pression négative et souple et sur lequel ladite conduite d'aspiration (10) est maintenue de manière étanche à la pression négative, dans lequel ledit moyen support (12) peut être appliqué, d'une manière pour l'essentiel étanche à la pression négative, sur le pansement à pression négative (6) recouvrant la plaie et fermant celle-ci de manière étanche par rapport à l'atmosphère, de sorte que la conduite d'aspiration (10) peut communiquer avec la zone de la plaie à travers au moins une ouverture (18) présente dans le moyen support (12) et dans le pansement à pression négative (6), dans lequel la conduite d'aspiration (10) est réalisée de manière souple et plate et est reliée au moyen support (12) dans une portion longitudinale (8) côté plaie, par au moins 70 % de sa surface projetée verticalement sur le moyen support (12), de manière inamovible et en nappe pour l'utilisation conforme à la destination, dans lequel ledit moyen support (12) et la conduite d'aspiration (10) sont parallèles entre eux avec leurs plans principaux résultant de leur forme plate, et dans lequel l'extension en épaisseur (D) de l'assemblage se composant de la conduite d'aspiration (10) et du moyen support (12) est de 7 mm tout au plus, **caractérisé par le fait qu'**une conduite d'alimentation en fluide (40) qui est menée au moyen support (12) dans le même plan que la conduite d'aspiration (10) et destinée à amener un milieu fluide à la zone de la plaie est prévue et que l'épaisseur de celle-ci n'est pas supérieure à l'épaisseur de la conduite d'aspiration (10), et que la conduite d'alimentation en fluide (40) passe à étanchement à travers une ouverture (42) présente dans le moyen support (12) et fait saillie de la face (22) du moyen support (12) laquelle montre vers la plaie, et peut se projeter dans la zone de la plaie et dans le pansement pour plaie (50) y prévue, à travers l'ouverture (20) présente dans le pansement à pression négative (6) et que, à cette fin, le pansement pour plaie (50) présente une ouverture de passage (54) qui s'étend dans la direction de profondeur de plaie, et que ladite ouverture de passage (54) dans le pansement pour plaie (50) est limitée par une douille d'insertion (56) dans laquelle la conduite d'alimentation en fluide (40) peut être introduite par sa portion (44) faisant saillie de la face (22) du moyen support (12) laquelle montre vers la plaie.

2. Ensemble matériel selon la revendication 1, **caractérisé par le fait que** la conduite d'alimentation en fluide (40) sort à un angle aigu, en particulier compris entre 30 et 70°, de la face (22) du moyen support (12) laquelle montre vers la plaie.

3. Ensemble matériel selon la revendication 1 ou 2, **caractérisé par le fait que**, sur une portion d'extrémité faisant saillie de la face (22) du moyen support (12) laquelle montre vers la plaie, la conduite d'alimentation en fluide (40) présente des ouvertures transversales (46) dans la paroi.

4. Ensemble matériel selon l'une ou plusieurs des revendications précédentes, **caractérisé par le fait que** la longueur de la portion de la conduite d'alimentation en fluide (40), qui fait saillie de la face (22) du moyen support (12) laquelle montre vers la plaie, est comprise entre 0,5 et 10 cm, en particulier entre 0,7 et 5 cm, en particulier entre 1 et 3 cm.

5. Ensemble matériel selon l'une ou plusieurs des revendications précédentes, **caractérisé par le fait que** sur la face (22) du moyen support (12), qui montre vers la plaie, est prévue une couche d'agent adhésif (24) ayant au moins trois couches qui comprend une couche porteuse centrale (26), une première couche de colle (28) maintenue sur ladite couche porteuse (26) et montrant vers le moyen support (12) ainsi qu'une deuxième couche de colle (30) maintenue sur la couche porteuse (26) et montrant dans la direction opposée au moyen support (12), et qui est réalisée de manière à ce qu'elle ne bloque pas ladite au moins une ouverture (18) dans le moyen support (12).

6. Ensemble matériel selon la revendication 5, **caractérisé par le fait que** la première couche de colle (28) et la deuxième couche de colle (30) sont formées de matières adhésives différentes ayant des propriétés de collage différentes.

7. Ensemble matériel selon la revendication 5 ou 6, **caractérisé par le fait que** le moyen support (12) est réalisé en silicone et que la première couche de colle (28) comprend une colle de silicone.

8. Ensemble matériel selon la revendication 6 ou 7, **caractérisé par le fait que** la deuxième couche de colle (30) comprend une colle d'acrylate.

9. Ensemble matériel selon l'une ou plusieurs des revendications précédentes 5 à 8, **caractérisé par le fait que** la surface adhésive de la première couche de colle (28) vers le moyen support (12) et/ou la surface adhésive de la deuxième couche de colle (30) vers le pansement à pression négative (6) est inférieure à 100 cm², en particulier inférieure à 50 cm², en particulier inférieure à 40 cm², en particulier inférieure à 30 cm².

10. Ensemble matériel selon l'une ou plusieurs des revendications précédentes 5 à 9, **caractérisé par le fait que** l'on utilise une couche d'agent adhésif (24) dont la première couche de colle (28) est réalisée de manière à ce que la couche d'agent adhésif (24) présente une force de pelage conférée par cette première couche de colle, par rapport à une plaque en acier, qui est comprise entre 25 et 40 N/100 mm, en particulier entre 27 et 35 N/100 mm, et/ou dont la deuxième couche de colle (30) est réalisée de manière à ce que la couche d'agent adhésif (24) présente une force de pelage conférée par cette deuxième couche de colle, par rapport à une plaque en acier, qui est comprise entre 70 et 85 N/100 mm, en particulier entre 70 et 80 N/100 mm.

11. Ensemble matériel selon l'une ou plusieurs des revendications précédentes 5 à 10, **caractérisé par le fait que** les première et deuxième couches de colle (28, 30) présentent la même extension en nappe en projection sur leur plan d'extension, et en particulier la même extension que la couche porteuse (26).

12. Ensemble matériel selon l'une ou plusieurs des revendications précédentes 5 à 11, **caractérisé par le fait que** les première et deuxième couches de colle (28, 30) présentent une épaisseur comprise entre 20 et 400 µm.

13. Ensemble matériel selon l'une ou plusieurs des revendications précédentes 5 à 12, **caractérisé par le fait que** la couche porteuse centrale (26) comprend une matière en non-tissé, une matière plate ayant une armure textile, comme par exemple un tricot ou tissu, ou une feuille en matière plastique, une feuille métallique ou un matériau composite en réalisé(e), ou en est constituée.

14. Ensemble matériel selon l'une ou plusieurs des revendications précédentes 5 à 13, **caractérisé par le fait que** la couche d'agent adhésif (24) s'étend sur l'ensemble de la face (22) du moyen support (12), qui montre vers la plaie.

15. Ensemble matériel selon l'une ou plusieurs des revendications précédentes 5 à 14, **caractérisé par le fait que** l'extension en nappe de ladite couche d'agent adhésif (24) fait au moins 50 % de la surface de la face (22) du moyen support (12), qui montre vers la plaie.
